# EUROPEAN PATENT SPECIFICATION

(11) **EP 1 443 931 B1**
(45) Date of publication and mention of the grant of the patent: **28.03.2007**
(21) Application number: 02752438.8
(22) Date of filing: 19.07.2002
(51) Int. Cl.: A61K 31/519, A61P 9/00, A61P 27/02, A61P 35/00, A61P 37/06, A61P 43/00, A61F 9/00, A61N 1/30, A61M 31/00, A61M 35/00

(54) **OCULAR IONTOPHORETIC DEVICE FOR DELIVERING METHOTREXATE BASED MEDICAMENTS AND USE THEREOF TO TREAT NEOPLASTIC, ANGIOGENIC, FIBROPLASTIC, AND/OR IMMUNOSUPPRESIVE OCULAR IRREGULARITIES**
IONTOPHORETISCHE OKULARVORRICHTUNG ZUR ABGABE VON MEDIKAMENTEN AUF METHOTREXAT-BASIS UND DEREN VERWENDUNG ZUR BEHANDLUNG VON NEOPLASTISCHEN, ANGIOGENEN, FIBROPLASTISCHEN UND/ODER IMMUNSUPPRESSIVEN AUGENUNREGELMÄSSIGKEITEN
DISPOSITIF IONTOPHORETIQUE OCULAIRE DESTINE A ADMINISTRER DES MEDICAMENTS A BASE DE METHOTREXATE ET UTILISATION DE CELUI-CI POUR TRAITER DES IRREGULARITES OCULAIRES NEOPLASIQUES, ANGIOGENIQUES, FIBROPLASTIQUES ET/OU IMMUNO-SUPPRESSIVES

(30) Priority: 20.07.2001 US 306789 P
(43) Date of publication of application: 11.08.2004
(73) Proprietor: IOMED, INC., Salt Lake City, Utah 84104 (US)
(72) Inventor: WARREN, Stephen, Fort Collins, CO 80528-8962 (US); HAMILTON, Steven, Park City, UT 84098 (US)
(74) Representative: Ostertag, Reinhard
(86) International application number: PCT/US2002/022861
(87) International publication number: WO 2003/007961

(56) References cited:
- EP-A- 0 299 467
- EP-A- 0 927 560
- WO-A-00/41730
- WO-A-00/51620
- WO-A-03/007961
- US-A- 4 515 794
- US-A- 5 487 895

## Description

### BACKGROUND OF THE INVENTION

### 1. Field of the Invention

The present invention relates in general to the use of a methotrexate based compound capable of inhibiting DNA synthesis in the manufacture of a medicament for treating an affected ocular area having neoplastic, angiogenic, fibroblastic, and/or immunosuppressive irregularity of a living subject. The present invention further relates to an ocular iontophoretic device for delivering a methotrexate based medicament to an affected area of a living subject's eye.

### 2. Background Art

Methotrexate based medicaments have been known in the art for years, and have been shown to possess anti-neoplastic, anti-angiogenic, anti-fibroblastic, and/or immunosuppressive activities. While administering methotrexate based medicaments have been identified as a promising remedy to treat many of the above-identified irregularities, delivering methotrexate based medicaments to an affected area of a living subject's eye has remained heretofore largely problematic. Indeed, known prior art methods of administering methotrexate based medicaments, identified hereinbelow, are replete with substantial drawbacks and/or life threatening complications.

For example, delivering methotrexate based medicaments to an affected, local area of a living subject's eye using a systemic delivery method is problematic because of the many severe, sometimes life threatening, side effects associated with systemic delivery of methotrexate based medicaments, such as, for examples, hepatitis, liver fibrosis, cirrhosis, leukopenia (bone marrow suppression), mucositis, ulcerative stomatitis, skin rash, nausea, abdominal distress, malaise, fatigue, chills and fever, diarrhea, gastrointestinal ulceration or perforation, pancreatitis, pericarditis, hypotension, deep venous thrombosis, thrombophlebitis, interstitial pneumonitis, headaches, drowsiness, cognitive dysfunction, reduced immunity, rash, photosensitivity, nephropathy, hematuria, alopecia, defective oogenesis, oligospermia, infertility, miscarriage, and birth defects.

Local delivery of methotrexate based medicaments via interocular injection remains problematic because of the opportunity for, among other things, retinal detachment, bleeding into the interior of the eye, increased interocular pressure, and increased risk of secondary infection. Although perhaps justifiable for occasional acute conditions, these risk factors render interocular injection undesirable as a delivery mode for anything less than critically acute ocular irregularities. Furthermore, interocular injections can not only be scary and unpleasant, but also extremely painful for the patient.

In addition to the above-identified problems associated with interocular injection, peribular or subconjuctival injection of methotrexate based medicaments can be problematic, because such injections may not deliver sufficient quantities to the interior of the eye. Moreover, peribular or subconjuctival injections are demanding of the physician inasmuch as placement of the needle requires an extremely high level of precision.

Topical administration of methotrexate based medicaments to an affected, local area of a living subject's eye is problematic due to its ineffectiveness for many applications, including affected areas in the back of the eye. An example for topical administration of a medicament is found in EP 0 299 467, wherein a methotrexate based medicament is instilled into the eye. In US 5 487 895, a methotrexate based medicament is applied to a living subject's eye using an ocular insert/bandage. However, for many applications such an insert/bandage may be ineffective. EP 0 927 560 A1 discloses a device for intraocular iontophoretic transfer of an active product. However, in the case of the delivery of a methotrexate based medicament to an affected area of a living subject's eye, a iontophoretic device has not been taken into consideration up to now.

### SUMMARY OF THE INVENTION

The present invention is directed to the use of a methotrexate based compound capable of inhibiting DNA synthesis in the manufacture of a medicament for treating an affected ocular area having neoplastic, angiogenic, fibroblastic, and/or immunosuppressive irregularity of a living subject, said medicament being adapted to be associated with the affected ocular area by an ocular iontophoretic device comprising an active electrode assembly associated with a matrix, wherein the matrix includes said medicament.

In a preferred embodiment of the present invention, the methotrexate based compound is represented by the following chemical structure: 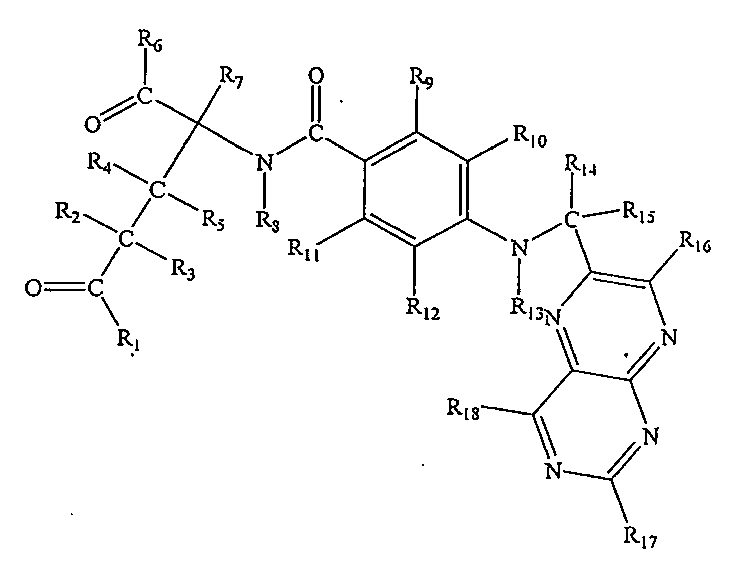 wherein R₁₋₁₈ are the same or different and comprise H, NH₂, a hydroxy group, a straight or branched alkyl, cycloalkyl, polycycloalkyl, heterocycloalkyl, aryl, alkaryl, aralkyl, alkoxy, alkenyl, alkynyl group containing 1 to 25 carbon atom(s), a silyl or siloxyl group containing 1 to 25 silicon atom(s), and combinations thereof. In this embodiment, the methotrexate based compound may comprise the structure:

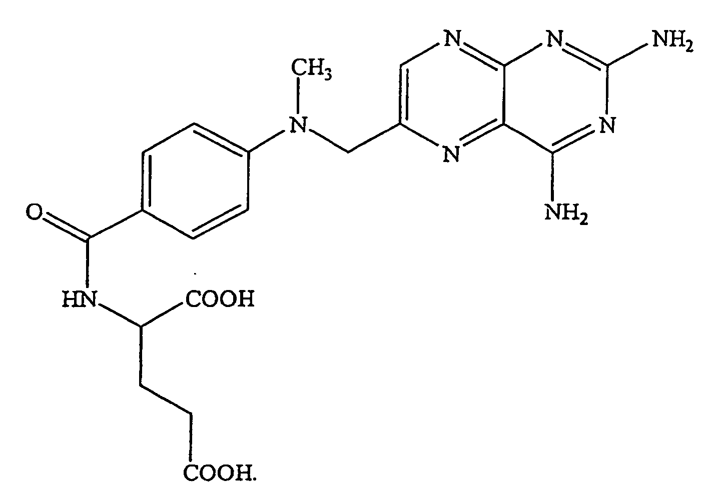

In another preferred embodiment of the present invention, the methotrexate based compound is 2-{4-[(2,4-Diamino-pteridin-6-ylmethyl)-methyl-amino]-benzoylamino}-pentanedioic acid and/or N-[4-[[(2,4-Diamino-6-pteridinyl)methyl]methylamino]-benzoyl]-L-glutamic acid and derivatives thereof.

In yet another preferred embodiment of the present invention, the medicament is adapted to an ocular iontophoretic delivery of the medicament in a concentration ranging from 0.5 to 50 mg/mL per day for 1 to 30 days.

Preferably, the ocular iontophoretic delivery of the medicament includes a delivery to at least one of the group consisting of the sclera, ciliary body, iris, lens, cornea, aqueous fluid, vitreous body, retina, choroids, optic nerve, and regions of the eye thereabout.

In accordance with the present invention, the ocular iontophoretic delivery of the medicament may include a delivery at a current between 0.5 mA and 5 mA for a period of between 1 and 60 minutes. Preferably, the ocular iontophoretic delivery of the medicament includes using negative polarity electrical current.

The present invention is further directed to an ocular iontophoretic device for delivering a methotrexate based medicament to an affected area of a living subject's eye, comprising an active electrode assembly associated with a matrix, wherein the matrix includes a methotrexate based medicament capable of decreasing neoplastic, angiogenic, fibroblastic, and/or immunosuppressive ocular irregularities of the living subject.

Further preferred embodiments of the ocular iontophoretic device are claimed in claims 11 to 20.

### BRIEF DESCRIPTION OF THE DRAWINGS

The invention will now be described with reference to the drawings wherein:
Fig. 1 of the drawings is a cross-sectional schematic representation of a first embodiment of an ocular iontophoretic device fabricated in accordance with the present invention;
Fig. 2 of the drawings is a cross-sectional schematic representation of a first embodiment of an ocular iontophoretic device fabricated in accordance with the present invention showing the association of a counter electrode assembly and an energy source; and
Fig. 3 of the drawings is a cross-sectional schematic representation of a second embodiment of an ocular iontophoretic device fabricated in accordance with the present invention.

### DETAILED DESCRIPTION OF THE INVENTION

While this invention is susceptible of embodiment in many different forms, there is shown in the drawings and will herein be described in detail several specific embodiments with the understanding that the present disclosure is to be considered as an exemplification of the principles of the invention.

It will be understood that like or analogous elements and/or components, referred to herein, may be identified throughout the drawings with like reference characters.

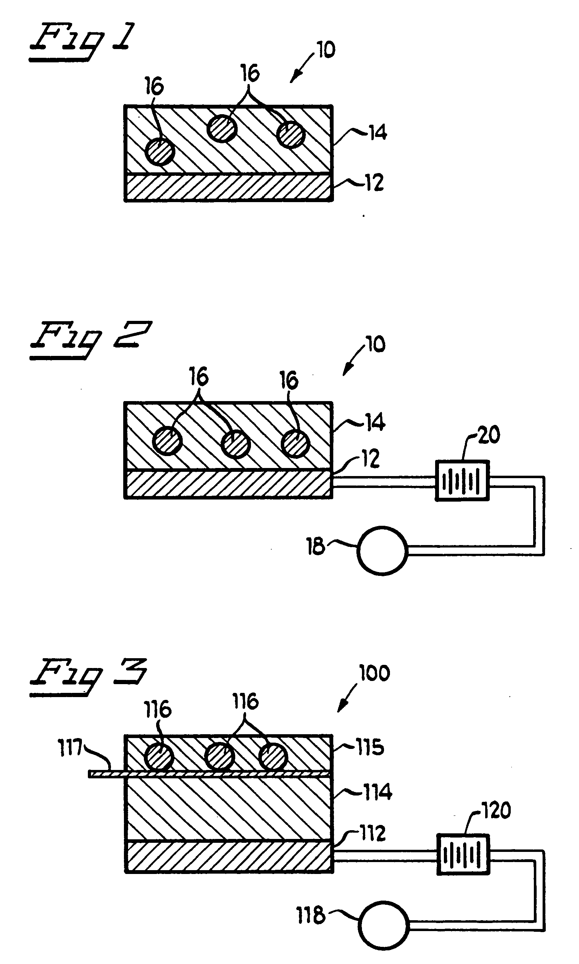

Referring now to the drawings and to Fig. 1 in particular, a first embodiment of an ocular iontophoretic device 10 is shown, which generally comprises active electrode assembly 12 and matrix 14. It will be understood that Fig. 1 is merely a cross-sectional schematic representation of ocular iontophoretic device 10. As such, some of the components have been distorted from their actual scale for pictorial clarity. As will be discussed in greater detail below, ocular iontophoretic device 10 is configured for delivering one or more methotrexate based medicament(s) which are capable of acting as an inhibitor of DNA, and, therefore, treating, among other things, neoplastic, angiogenic, fibroblastic, and/or immunosuppressive ocular irregularities. By iontophoretically administering a methotrexate based medicament to an affected area of a living subject's eye, diseases associated with the above-identified ocular irregularities can be efficiently remedied - especially including diseases of the eye wherein the affected area is toward the back of the eye, or generally proximate the optic nerve. Moreover, by utilizing iontophoretic technology, the living subject does not need to be exposed to such high medicament concentrations, which is of particular importance with such a potent classification of medicaments, because toxicity build can occur rapidly using conventional, for example, systemic administration methods. Ocular iontophoretic device 10 offers many advantages over the previously discussed prior art devices and associated delivery methods, including, but not limited to, simultaneous enablement of non-invasive and deep methotrexate based medicament delivery, non-invasive local delivery of an effective, therapeutic level of methotrexate based medicament while minimizing systemic concentrations, and enablement of, for example, sclera loading for prolonged delivery (of controlled, sometimes, low concentrations of medicaments) into regions in the back of the eye.

Active electrode assembly 12 generally comprises a conductive material, which upon application of an electrical potential difference thereto, drives an ionic methotrexate based medicament (i.e. an anionic medicament), received from matrix 14 and delivers the methotrexate based medicament into predetermined tissues and surrounding structures of a living subject's eye. It will be understood that active electrode assembly 12 may comprise an anode or a cathode depending upon whether the medicament is cationic or anionic in form. It will be further understood that active electrode assembly may include an open-faced or high current density electrode. As would be readily understood to those having ordinary skill in the art, any one of a number of conventional active electrode assemblies are contemplated for use in accordance with the present invention. The only contemplated limitation relative to active electrode assembly 12 is that it must be geometrically and compositionally compatible for ocular applications of living subjects, most relevantly, humans.

Matrix 14 extends contiguously from active electrode 12, and is preferably fabricated from a material capable of temporarily retaining methotrexate based medicament 16 in solution. The solution may also contain supplemental agents, such as electrolytes, stability additives, medicament preserving additives, pH regulating buffers, etc. Matrix 14 may comprise, for example, a natural or synthetic amorphous member, a natural or synthetic sponge pad, a natural or synthetic lint free pad, a natural or synthetic low particulate member - just to name a few. Indeed, numerous other materials that would be known to those having ordinary skill in the art having the present disclosure before them are likewise contemplated for use. As with active electrode assembly 12, the only contemplated limitation relative to matrix 14 is that it must be geometrically and compositionally compatible for ocular applications of living beings, most relevantly, humans.

Medicament 16 is retained within matrix 14. In accordance with the present invention, ionic medicament 16 comprises one or more methotrexate based medicament(s) which are capable of treating, among other things, neoplastic, angiogenic, fibroblastic, and/or immunosuppressive ocular irregularities.

Such methotrexate based medicaments may be represented by the following chemical structure: 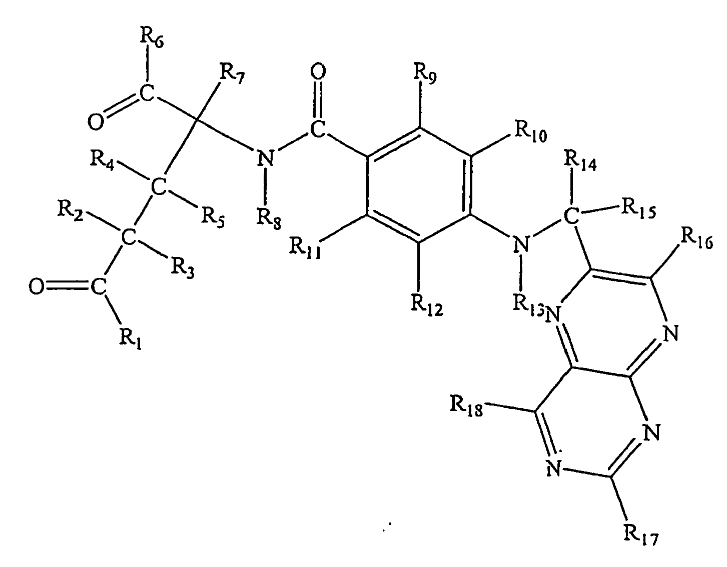 wherein R₁₋₁₈ are the same or different and comprise H, NH₂, a hydroxy group, a straight or branched alkyl, cycloalkyl, polycycloalkyl, heterocycloalkyl, aryl, alkaryl, aralkyl, alkoxy, alkenyl, alkynyl group containing 1 to 25 carbon atom(s), a silyl or siloxyl group containing 1 to 25 silicon atom(s), and combinations thereof, and the pharmaceutically acceptable acid addition salts thereof. It will be understood that the availability of methotrexate medicaments will be readily known to those having ordinary skill in the art (such as those sold under the trade name FOLEX and MEXATE), and that derivatives thereof may be obtained using conventional organic synthetic routes.

For example, the methotrexate based medicament may comprise the structure:

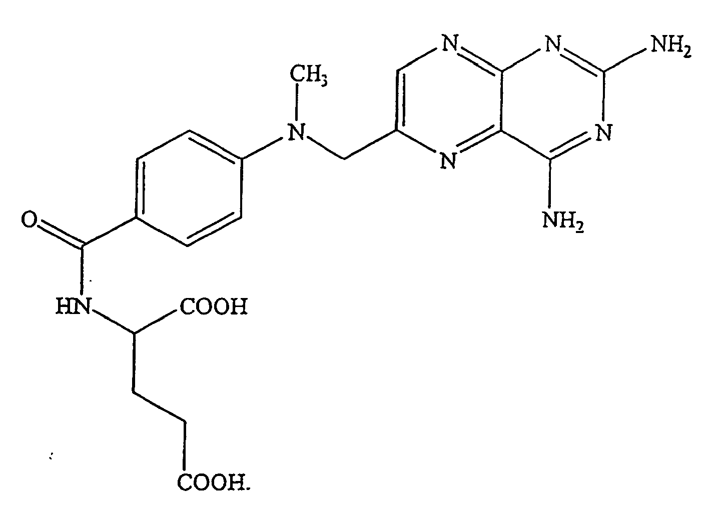

In a preferred embodiment of the present invention, the methotrexate based medicaments may include 2-{4-[(2,4-Diamino-pteridin-6-ylmethyl)-methyl-amino]-benzoylamino}-pentanedioic acid and/or N-[4-[[(2,4-Diamino-6-pteridinyl)methyl] methylamino]benzoyl]-L-glutamic acid and derivatives thereof.

As is shown in Fig. 2, ocular iontophoretic device 10 may also include counter electrode assembly 18 and energy source 20. Counter electrode assembly 18 may be housed within ocular iontophoretic device 10, or alternatively, may be remotely associated with ocular iontophoretic device 10 via conventional electrical conduit. Counter electrode assembly 18 is configured for completing an electrical circuit between active electrode assembly 12 and energy source 20. As with active electrode 12, counter electrode 18 may comprise an anode or a cathode depending upon whether the medicament is cationic or anionic in form. As would be readily understood to those having ordinary skill in the art, any one of a number of counter electrodes are contemplated for use in accordance with the present invention.

Similarly to counter electrode assembly 18, energy source 20 may be housed within ocular iontophoretic device 10, or alternatively, may be remotely associated with ocular iontophoretic device 10 via conventional electrical conduit. Energy source 20 preferably supplies low voltage constant direct current between 0.5 milliamps (mA) and 5 mA for generating an electrical potential difference. The energy source may also provide for an initial higher voltage during current ramp-up to break down higher initial tissue resistance as in commercial power supply units used for transdermal iontophoresis. For purposes of the present disclosure, energy source 20 may include one or more primary or secondary electrochemical cells. While specific examples of energy source 20 have been disclosed, for illustrative purposes only, it will be understood that other energy sources known to those having ordinary skill in the art having the present disclosure before them are likewise contemplated for use.

Referring now to the drawings and to Fig. 3 in particular, a second embodiment of an ocular iontophoretic device 100 is shown, which generally comprises active electrode assembly 112, matrix 114, reservoir 115, counter electrode assembly 118, and energy source 120. It will be understood that active electrode assembly 112, matrix 114, counter electrode assembly 118, and energy source 120, are configured analogously to previously discussed active electrode assembly 12, matrix 14, counter electrode assembly 18, and energy source 20, respectively. Ocular iontophoretic device 100 is configured for delivering a methotrexate based medicament to an affected area of a living subject's eye for treating neoplastic, angiogenic, fibroblastic, and/or immunosuppressive ocular irregularities.

Reservoir 115 includes methotrexate based medicament 116, in solution, which is capable of treating the above-identified ocular irregularities. Reservoir 115 may include a releasable cover member 117 which, upon articulation, releases methotrexate based medicament 116 into matrix 114. Such a release cover enables prompt delivery of the methotrexate based medicament with very little device preparation.

When an affected area of a living subject's eye shall be treated, the following steps may be carried out: First, a methotrexate based medicament is associated with an ocular iontophoretic device. Preferably, the methotrexate based medicament is metered from a syringe or single unit dose. Second, at least a portion of the ocular iontophoretic device is positioned on the eye of a living being. Finally, the methotrexate based medicament is iontophoretically delivered to an affected area of the living subject's eye. Preferably, the delivery lasts for between 1 and 60 minutes. Compared to prior art administration or delivery methods, the present invention enables a generally painless, non-invasive, and deep delivery of the methotrexate based medicament. Moreover, the methotrexate based medicament is locally delivered to an affected area of a living subject's eye at an effective, therapeutic level. Preferred ocular delivery regions include the sclera, ciliary body, iris, lens, cornea, aqueous fluid, vitreous body, retina, choroids, optic nerve, and regions of the eye thereabout.

For purposes of the present disclosure, neoplastic, angiogenic, fibroblastic, and/or immunosuppressive ocular irregularities of a living subject can also be treated in accordance with the following method. First, a living subject with a neoplastic, angiogenic, fibroblastic, and/or immunosuppressive irregularity is provided. Second, one or more of the above-identified methotrexate based medicaments is provided. Third, a therapeutically effective concentration of the methotrexate based medicament is associated with and/or administered to the affected ocular area of the living subject. Preferably, the methotrexate based medicament is administered in a concentration ranging from 0.5 to 50 mg/mL. The duration of a single application may range from 1 minute to 60 minutes. The medicament may be administered on a schedule ranging from once every day to once every 30 days. The duration of methotrexate based therapy may range from a single application to multiple applications that are administered over a period of months to years, depending upon the disease being treated. Upon administration of the methotrexate based medicament, the neoplastic, angiogenic, fibroblastic, and/or immunosuppressive ocular irregularity of the living subject is materially decreased.

Methotrexate is dissolved in a balanced saline solution, for example, sodium chloride (e.g. 0.25 to 0.9% w/v). The solution may be buffered with other salts, such as phosphate, carbonate, or citrate. The pH is adjusted to a value between 4.0 and 9.0, preferably pH 7.5, using NaOH or HCl. The final concentration of methotrexate is between 0.5 and 50 mg/mL. Iontophoretic current is applied at 1.0 to 4.0 milliamperes for 1 to 60 minutes. It will be understood to those having ordinary skill in the art that the previously identified formulation, although being preferred, is not the only formulation which can be used.

## Claims

1. Use of a methotrexate based compound capable of inhibiting DNA synthesis in the manufacture of a medicament for treating an affected ocular area having neoplastic, angiogenic, fibroblastic, and/or immunosuppressive irregularity of a living subject said medicament being adapted to be associated with the affected ocular area by an ocular iontophoretic device comprising an active electrode assembly associated with a matrix, wherein the matrix includes said medicament.

2. The use according to claim 1, wherein the methotrexate based compound is represented by the following chemical structure: 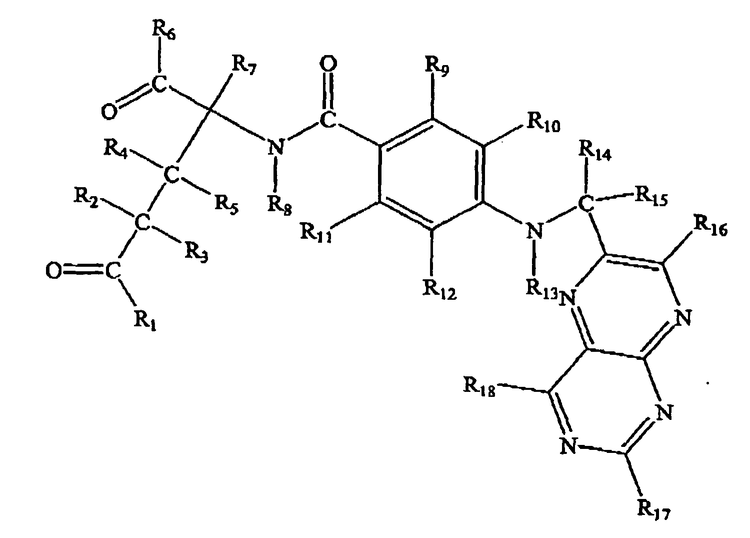 wherein R₁₋₁₈ are the same or different and comprise H, NH₂, a hydroxy group, a straight or branched alkyl, cycloalkyl, polycycloalkyl, heterocycloalkyl, aryl, alkaryl, aralkyl, alkoxy, alkenyl, alkynyl group containing 1 to 25 carbon atom(s), a silyl or siloxyl group containing 1 to 25 silicon atom(s), and combinations thereof.

3. The use according to claim 1, wherein the methotrexate based compound is represented by the following chemical structure:

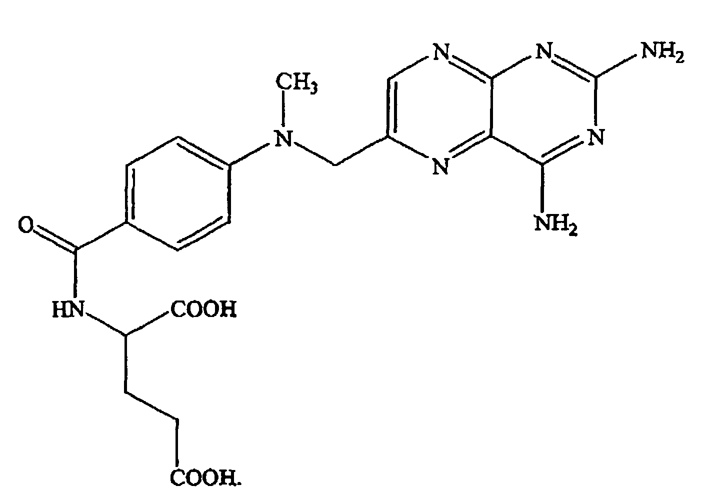

4. The use according to claim 1, wherein the methotrexate based compound is 2-{4-[(2,4-Diamino-pteridin-6-ylmethyl)-methyl-amino]-benzoylamino}-pentanedioic acid and derivatives thereof.

5. The use according to claim 1, wherein the methotrexate based compound is N-[4-[[(2,4-Diamino-6-pteridinyl)methyl]methylamino]benzoyl]-L-glutamic acid and derivatives thereof.

6. The use according to any of claims 1 to 5, wherein the medicament is adapted to an ocular iontophoretic delivery of the medicament in a concentration ranging from 0.5 to 50 mg/mL. per day for 1 to 30 days.

7. The use according to claim 6 wherein the ocular iontophoretic delivery of the medicament includes a delivery to at least one of the group consisting of the sclera, ciliary body, iris, lens, cornea, aqueous fluid, vitreous body, retina, choroids, optic nerve, and regions of the eye thereabout.

8. The use according to claim 6 or 7, wherein the ocular iontophoretic delivery of the medicament includes a delivery at a current between 0.5 mA and 5 mA for a period of between 1 and 60 minutes.

9. The use according to any of claims 6 to 8, wherein the ocular iontophoretic delivery of the medicament includes using negative polarity electrical current.

10. An ocular iontophoretic device for delivering a methotrexate based medicament to an affected area of a living subject's eye, comprising:
- an active electrode assembly associated with a matrix, wherein the matrix includes a methotrexate based medicament according to claim 1.

11. An ocular iontophoretic device according to claim 10
wherein the active electrode assembly is configured for iontophoretically delivering tha methotrexate based medicament to the affected area of the living subject's eye; further comprising
- a counter electrode assembly, wherein the counter electrode assembly is configured for completing an electrical circuit between the active electrode assembly and an energy source; and
- an energy source for generating an electrical potential difference.

12. The ocular iontophoretic device according to claim 10 or 11, wherein the affected area of the living subject's eye is selected from at least one of the group consisting of the sclera, ciliary body, iris, less, cornea, aqueous fluid, vitreous body, retina, choroids, optic nerve, and regions of the eye thereabout.

13. The ocular iontophoretic device according any of claims 10 to 12, wherein the active electrode assembly includes an open-faced or high current density electrode.

14. The ocular iontophoretic device according any of claims 10 to 13, wherein the methotrexate based medicament is represented by the following chemical structure: 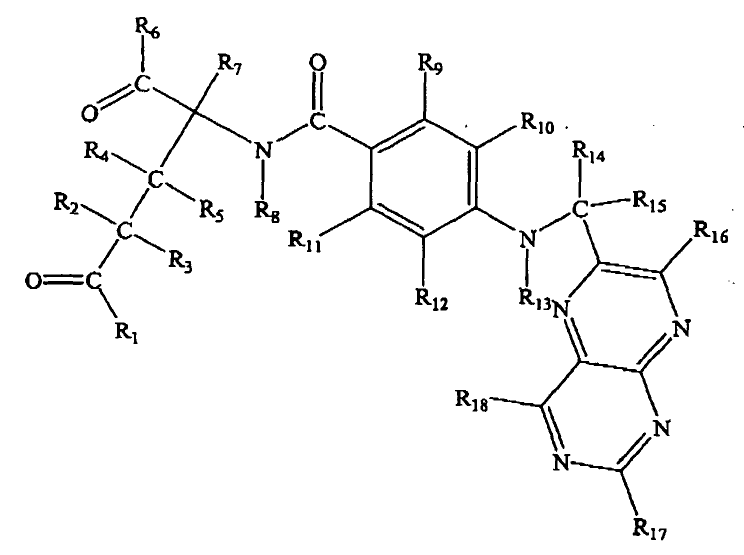 wherein R₁₋₁₈ are the same or different and comprise H, NH₂, a hydroxy group, a straight or branched alkyl, cycloalkyl, polycycloalkyl, heterocycloalkyl, aryl, alkaryl, aralkyl, alkoxy, alkenyl, alkynyl group containing 1 to 25 carbon atom(s), a silyl or siloxyl group containing 1 to 25 silicon atom(s), and combinations thereof.

15. The ocular iontophoretic device according to any claim 10 to 13, wherein the methotrexate based medicament is represented by the following chemical structure:

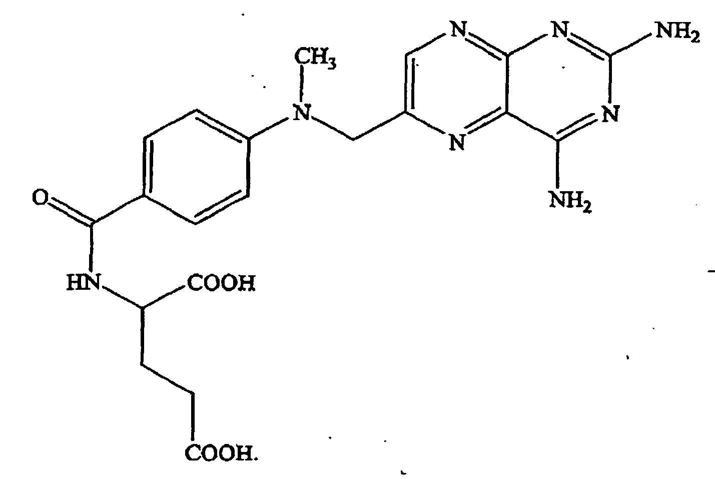

16. The ocular iontophoretic device according to any of claims 10 to 13, wherein the methotrexate based medicament comprises 2-{4-[(2,4-Diamino-pteridin-6-ylmethyl)-methyl-amino]-benzoyl-amino}-pentanedioic acid and derivatives thereof.

17. The ocular iontophoretic device according to any of claim 10 to 13, wherein the methotrexate based medicament comprises N-[4-[[(2,4-Diamino-6-pteridinyl)methyl] methylamino]-benzoyl]-L-glutamic acid and derivatives thereof.

18. An ocular iontophoretic device according to any of claims 11 to 17, further comprising
- a reservoir, wherein the reservoir includes said methotrexate based medicament;
and wherein the matrix is capable of temporarily retaining a solution having said methotrexate based medicament;

19. The ocular iontophoretic device according to any of claims 10 to 18, wherein the methotrexate based medicament is formulated in a 0.5 mg/mL. compound and 50 mg/mL compound buffer.

20. The ocular iontophoretic device according to claim 19, wherein the buffer ranges in pH from 4.0 to 9.0, and, preferably pH 7.5.

## Patentansprüche

1. Verwendung einer Verbindung auf Methotrexat-Basis, welche die DNA-Synthese hemmen kann, bei der Herstellung eines Medikaments zur Behandlung eines betroffenen Augenbereichs eines lebenden Subjekts mit einer neoplastischen, angiogenetischen, fibroblastischen und/oder immunsuppressiven Unregelmäßigkeit, wobei das Medikament so angepasst ist, dass es mit dem betroffenen Augenbereich mittels einer Augen-lontophoresevorrichtung in Verbindung gebracht wird, welche eine aktive Elektrodenanordnung umfasst, die mit einer Matrix assoziiert ist, wobei die Matrix das Medikament einschließt.

2. Verwendung nach Anspruch 1, bei der die Verbindung auf Methotrexat-Basis durch die folgende chemische Struktur dargestellt wird: 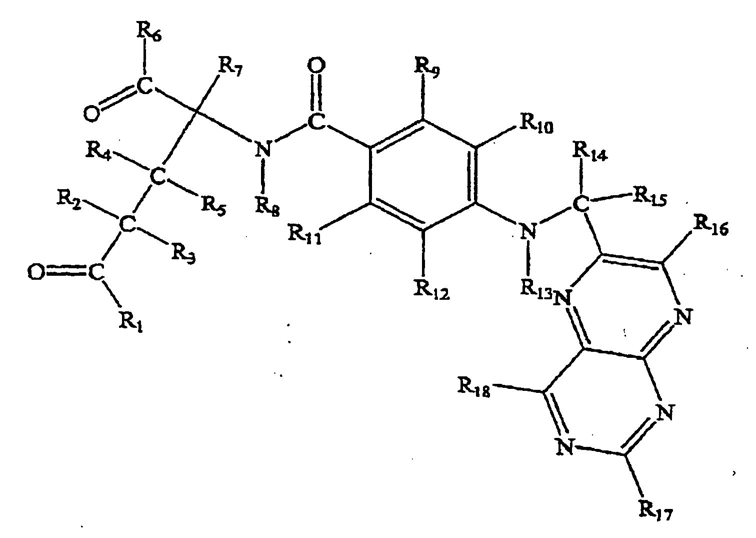 in der R₁₋₁₈ gleich oder verschieden sind und H, NH₂, eine Hydroxygruppe, eine gerade oder verzweigte Alkyl-, Cycloalkyl-, Polycycloalkyl-, Heterocycloalkyl-, Aryl-, Alkaryl-, Aralkyl-, Alkoxy-, Alkenyl-, Alkinylgruppe, die 1 bis 25 Kohlenstoffatom(e) enthält, eine Silyl- oder Siloxylgruppe, die 1 bis 25 Siliciumatom(e) enthält, und deren Kombinationen umfassen.

3. Verwendung nach Anspruch 1, bei der die Verbindung auf Methotrexat-Basis durch die folgende chemische Struktur dargestellt wird:

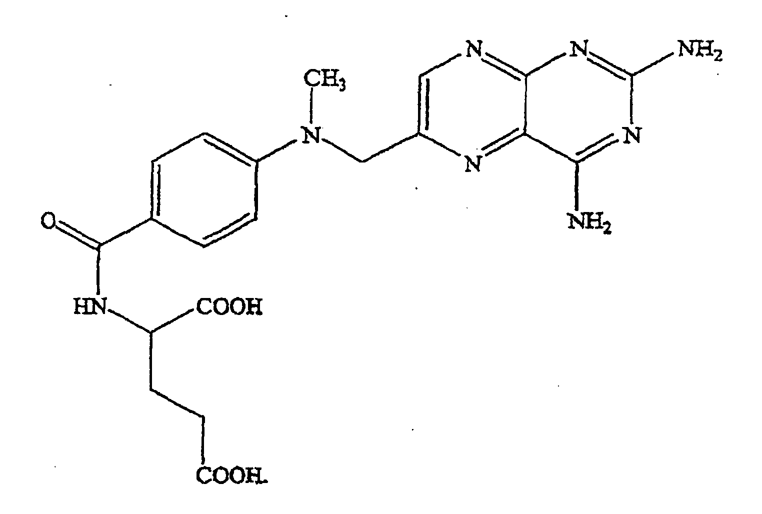

4. Verwendung nach Anspruch 1, bei der es sich bei der Verbindung auf Methotrexat-Basis um 2-4-{(2,4-Diaminopteridin-6-ylmethyl)methylamino]-benzoylamino}pentandisäure und Derivate derselben handelt.

5. Verwendung nach Anspruch 1, bei der es sich bei der Verbindung auf Methotrexat-Basis um N-4-[[(2,4-Diamino-6-pteridinyl)methyl]methylamino]-benzoyl]-L-glutaminsäure und Derivate derselben handelt.

6. Verwendung nach irgendeinem der Ansprüche 1 bis 5, bei der das Medikament an eine Augen-lontophoresezufuhr des Medikaments in einer Konzentration im Bereich von 0,5 bis 50 mg/ml pro Tag über 1 bis 30 Tage angepasst ist.

7. Verwendung nach Anspruch 6, bei der die Augen-lontophoresezufuhr des Medikaments eine Zufuhr zu mindestens einem aus der Gruppe einschließt, die aus Sklera, Ziliarkörper, Iris, Linse, Kornea, wässrigem Fluid, Glaskörper, Retina, Choroidea, Sehnerv und Bereichen des Auges darum herum besteht.

8. Verwendung nach Anspruch 6 oder 7, bei der die Augen-lontophoresezufuhr des Medikaments eine Zufuhr bei einem Strom zwischen 0,5 mA und 5 mA über eine Zeitspanne zwischen 1 und 60 Minuten umfasst.

9. Verwendung nach irgendeinem der Ansprüche 6 bis 8, bei der die Augen-lontophoresezufuhr des Medikaments die Verwendung eines elektrischen Stroms mit negativer Polarität einschließt.

10. Augen-lontophoresevorrichtung für die Zufuhr eines Medikaments auf Methotrexat-Basis zu einem betroffenen Bereich eines Auges eines lebenden Subjekts, umfassend:
- eine aktive Elektrodenanordnung, die mit einer Matrix verbunden ist, wobei die Matrix ein Medikament auf Methotrexat-Basis nach Anspruch 1 einschließt.

11. Augen-lontophoresevorrichtung nach Anspruch 10
bei der die aktive Elektrodenanordnung für eine iontophoretische Zufuhr des Medikaments auf Methotrexat-Basis an den betroffenen Bereich des Auges des lebenden Subjekts konfiguriert ist; weiter umfassend
- eine Gegenelektrodenanordnung, bei der Gegenelektrodenanordnung zum Schließen eines elektrischen Stromkreises zwischen der aktiven Elektrodenanordnung und einer Energiequelle konfiguriert ist; und
- eine Energiequelle zum Erzeugen eines elektrischen Potentialunterschieds.

12. Augen-lontophoresevorrichtung nach Anspruch 10 oder 11, bei der der betroffene Bereich des Auges des lebenden Subjekts ausgewählt ist aus mindestens einem der Gruppe bestehend aus Sklera, Ziliarkörper, Iris, Linse, Kornea, wässrigem Fluid, Glaskörper, Retina, Choroidea, Sehnerv und Bereichen des Auge darum herum.

13. Augen-Iontophoresevorrichtung nach irgendeinem der Ansprüche 10 bis 12, bei der die aktive Elektrodenanordnung eine Elektrode mit offener Fläche oder hoher Stromdichte einschließt.

14. Augen-lontophoresevorrichtung nach irgendeinem der Ansprüche 10 bis 13, in der das Medikament auf Methotrexat-Basis durch die folgende chemische Struktur dargestellt wird: 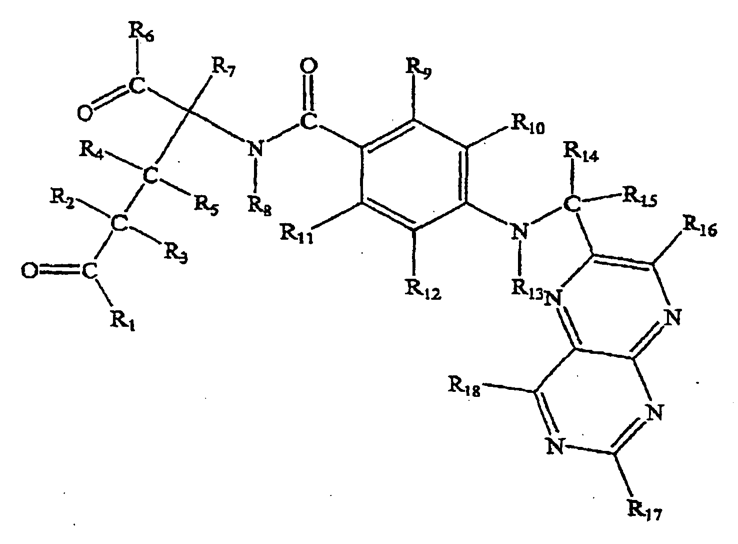 in der R₁₋₁₈ gleich oder verschieden sind und H, NH₂, eine Hydroxygruppe, eine gerade oder verzweigte Alkyl-, Cycloalkyl-, Polycycloalkyl-, Heterocycloalkyl-, Aryl-, Alkaryl-, Aralkyl-, Alkoxy-, Alkenyl-, Alkinylgruppe, die 1 bis 25 Kohlenstoffatom(e) enthält, eine Silyl- oder Siloxylgruppe, die 1 bis 25 Siliciumatom(e) enthält, und deren Kombinationen umfassen.

15. Augen-lontophoresevorrichtung nach irgendeinem der Ansprüche 10 bis 13, in der das Medikament auf Methotrexat-Basis durch die folgende chemische Struktur dargestellt wird:

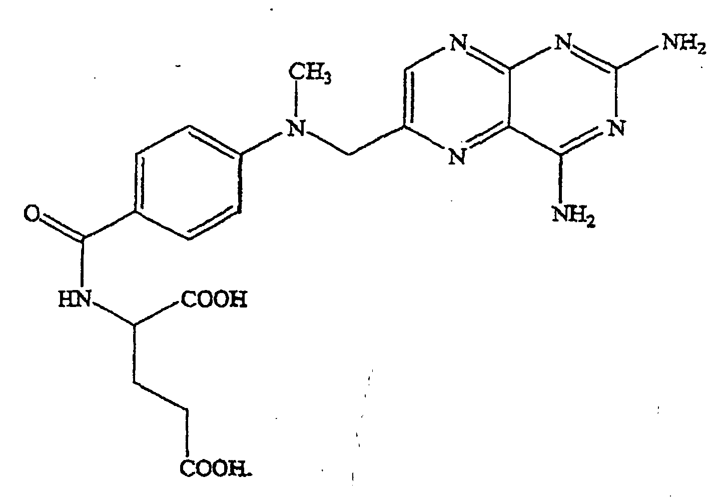

16. Augen-lontophoresevorrichtung nach irgendeinem der Ansprüche 10 bis 13, in der es sich bei dem Medikament auf Methotrexat-Basis um 2- {4-[(2,4-Diaminopteridin-6-ylmethyl)methylamino]benzoylamino}pentandisäure und Derivate derselben handelt.

17. Augen-lontophoresevorrichtung nach irgendeinem der Ansprüche 10 bis 13, in der es sich bei dem Medikament auf Methotrexat-Basis um N-4-[[(2,4-Diamino-6-pteridinyl)methyl]methylamino]benzoyl]-L-glutaminsäure und Derivate derselben handelt.

18. Augen-lontophoresevorrichtung nach irgendeinem der Ansprüche 11 bis 17, weiter umfassend:
- ein Reservoir, wobei das Reservoir das Medikament auf Methotrexat-Basis einschließt,
und wobei die Matrix in der Lage ist, vorübergehend eine Lösung mit dem Medikament auf Methotrexat-Basis zu halten.

19. Augen-lontophoresevorrichtung nach irgendeinem der Ansprüche 10 bis 18, in der das Medikament auf Methotrexat-Basis als 0,5 mg/ml Verbindung und 50 mg/ml Verbindungspuffer formuliert ist.

20. Augen-lontophoresevorrichtung nach Anspruch 19, in der der Puffer einen pH im Bereich von 4,0 bis 9,0 und bevorzugt von pH 7,5 aufweist.

## Revendications

1. Utilisation d'un composé à base de méthotrexate pouvant ou apte à inhiber la synthèse d'ADN dans la fabrication d'un médicament destiné à traiter une zone oculaire affectée possédant une irrégularité néoplastique, angiogénique, fibroblastique, et/ou immunosuppressive d'un sujet vivant, ledit médicament étant adapté pour être associé à la zone oculaire affectée par un dispositif iontophorétique oculaire comprenant un ensemble d'électrode actif associé à une matrice, la matrice comprenant ledit médicament.

2. Utilisation selon la revendication 1, dans laquelle le composé à base de méthotrexate est représenté par la structure chimique suivante : 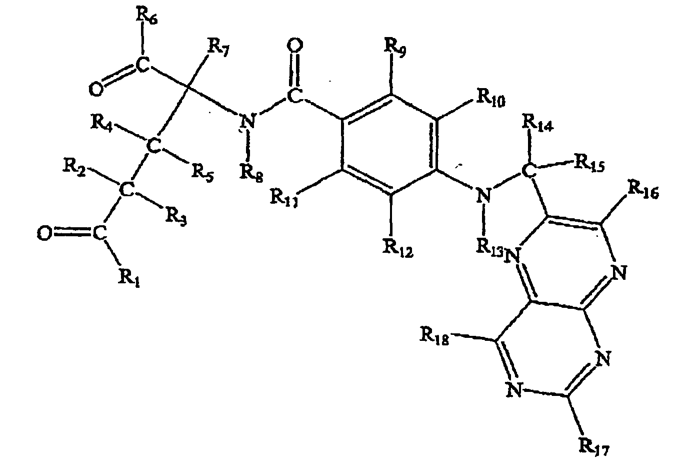 où les groupements R_{1 à 18} sont identiques ou différents et comprennent H, NH₂, un groupe hydroxy, un groupe alkyle, cycloalkyle, polycycloalkyle, hétérocycloalkyle, aryle, alkaryle, aralkyle, alcoxy, alcényle, alcynyle linéaire ou ramifié contenant de 1 à 25 atomes de carbone, un groupe silyl ou siloxyl contenant de 1 à 25 atomes de silicium et des combinaisons de ceux-ci.

3. Utilisation selon la revendication 1, dans laquelle le composé à base de méthotrexate est représenté par la structure chimique suivante :

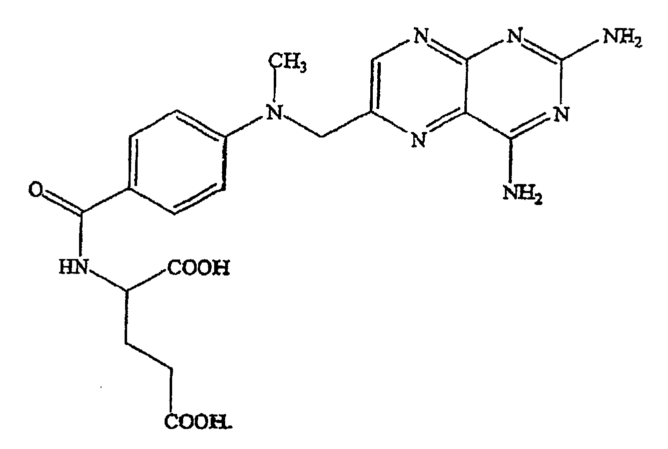

4. Utilisation selon la revendication 1, dans laquelle le composé à base de méthotrexate est l'acide 2-{4-[(2,4-diamino-pteridin-6-ylméthyl)-méthyl-amino]-benzoylamino}pentanedioïque et ses dérivés.

5. Utilisation selon la revendication 1, dans laquelle le composé à base de méthotrexate est l'acide N-[4-[[(2,4-diamino-6-pteridinyl)méthyl]méthylamino]benzoyl]-L-glutamique et ses dérivés.

6. Utilisation selon l'une quelconque des revendications 1 à 5, dans laquelle le médicament est approprié à une délivrance iontophorétique oculaire du médicament dans une gamme de concentrations allant de 0,5 à 50 mg/mL par jour pendant 1 à 30 jours.

7. Utilisation selon la revendication 6, dans laquelle la délivrance iontophorétique oculaire du médicament comprend une délivrance d'au moins un élément du groupe constitué de la sclère, du corps ciliaire, de l'iris, du cristallin, de la cornée, du fluide aqueux, du corps vitreux, de la rétine, des choroïdes, du nerf optique et des régions aux alentours de l'oeil.

8. Utilisation selon la revendication 6 ou 7, dans laquelle la délivrance iontophorétique oculaire du médicament comprend une délivrance à ou sous un courant compris entre 0,5 mA et 5 mA pendant une période comprise entre 1 et 60 minutes.

9. Utilisation selon l'une quelconque des revendications 6 à 8, dans laquelle la délivrance iontophorétique oculaire du médicament comprend l'utilisation d'un courant électrique de polarité négative.

10. Dispositif iontophorétique oculaire destiné à délivrer un médicament à base de méthotrexate à une zone affectée de l'oeil d'un sujet vivant, comprenant :
- un ensemble d'électrode actif associé à une matrice, dans lequel la matrice comprend un médicament à base de méthotrexate conformément à la revendication 1.

11. Dispositif iontophorétique oculaire selon la revendication 10
dans lequel l'ensemble d'électrode actif est configuré pour délivrer de façon iontophorétique le médicament à base de méthotrexate à la zone affectée de l'oeil du sujet vivant, comprenant en outre
- un ensemble de contre-électrode, dans lequel l'ensemble de contre-électrode est configuré pour compléter un circuit électrique entre l'ensemble d'électrode actif et une source d'énergie, et
- une source d'énergie destinée à générer une différence de potentiel électrique.

12. Dispositif iontophorétique oculaire selon la revendication 10 ou 11, dans lequel la zone affectée de l'oeil du sujet vivant est sélectionnée à partir d'au moins un élément du groupe constitué de la sclère, du corps ciliaire, de l'iris, du cristallin, de la cornée, du fluide aqueux, du corps vitreux, de la rétine, des choroïdes, du nerf optique et des régions aux alentours de l'oeil.

13. Dispositif iontophorétique oculaire selon l'une quelconque des revendications 10 à 12, dans lequel l'ensemble d'électrode actif comprend une électrode de densité de courant élevée ou à face ouverte.

14. Dispositif iontophorétique oculaire selon l'une quelconque des revendications 10 à 13, dans lequel le médicament à base de méthotrexate est représenté par la structure chimique suivante : 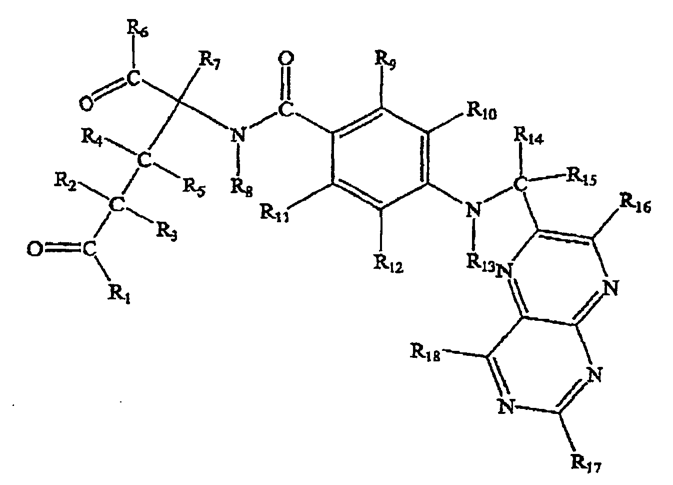 où les groupements R_{1 à 18} sont identiques ou différents et comprennent H, NH₂, un groupe hydroxy, un groupe alkyle, cycloalkyle, polycycloalkyle, hétérocycloalkyle, aryle, alkaryle, aralkyle, alcoxy, alcényle, alcynyle linéaire ou ramifié contenant de 1 à 25 atomes de carbone, un groupe silyl ou siloxyl contenant de 1 à 25 atomes de silicium et des combinaisons de ceux-ci.

15. Dispositif iontophorétique oculaire selon l'une quelconque des revendications 10 à 13, dans lequel le médicament à base de méthotrexate est représenté par la structure chimique suivante :

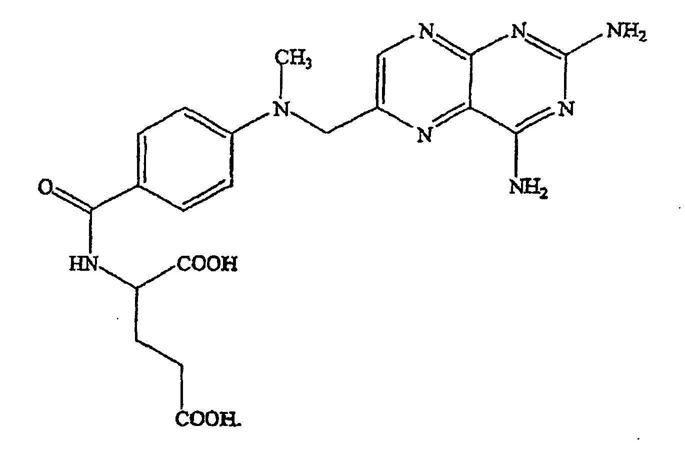

16. Dispositif iontophorétique oculaire selon l'une quelconque des revendications 10 à 13, dans lequel le médicament de méthotrexate comprend l'acide 2-{4-[(2,4-diamino-pteridin-6-ylméthyl)-méthyl-amino]-benzoyl-amino}pentanedioïque et ses dérivés.

17. Dispositif iontophorétique oculaire selon l'une quelconque des revendications 10 à 13, dans lequel le médicament à base de méthotrexate comprend l'acide N-[4-[[(2,4-diamino-6-pteridinyl)méthyl]méthylamino]-benzoyl]-L glutamique et ses dérivés.

18. Dispositif iontophorétique oculaire selon l'une quelconque des revendications 11 à 17 comprenant en outre
- un réservoir dans lequel le réservoir comprend ledit médicament à base de méthotrexate,
et dans lequel la matrice est capable de retenir de façon temporaire une solution présentant ledit médicament à base de méthotrexate.

19. Dispositif iontophorétique oculaire selon l'une quelconque des revendications 10 à 18, dans lequel le médicament à base de méthotrexate est formulé de 0,5 mg/mL du composé et de 50 mg/mL de composé tampon.

20. Dispositif iontophorétique oculaire selon la revendication 19, dans lequel le tampon a un pH dans la gamme de 4,0 à 9,0 et de préférence un pH égal à 7,5.
